(19) Europäisches Patentamt
European Patent Office
Office européen des brevets

(11) **EP 2 962 635 A1**

(12) **EUROPEAN PATENT APPLICATION**
published in accordance with Art. 153(4) EPC

(43) Date of publication:
06.01.2016 Bulletin 2016/01

(51) Int Cl.:
*A61B 5/0484* (2006.01)

(21) Application number: 14756336.5

(22) Date of filing: 25.02.2014

(86) International application number:
PCT/JP2014/054559

(87) International publication number:
WO 2014/132964 (04.09.2014 Gazette 2014/36)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: 27.02.2013 JP 2013038005

(71) Applicant: **Toyota Jidosha Kabushiki Kaisha Toyota-shi, Aichi 471-8571 (JP)**

(72) Inventors:
• **KITAJO, Keiichi**
  **Wako-shi**
  **Saitama 351-0198 (JP)**
• **YAMADA, Hitoshi**
  **Toyota-shi**
  **Aichi 471-8571 (JP)**
• **KOSAKA, Yusuke**
  **Toyota-shi**
  **Aichi 471-8571 (JP)**

(74) Representative: **TBK**
  **Bavariaring 4-6**
  **80336 München (DE)**

(54) **BRAIN WAVE SIGNAL PROCESSING DEVICE, BRAIN WAVE SIGNAL PROCESSING METHOD, PROGRAM, AND RECORDING MEDIUM**

(57)    The present invention includes (i) a right instantaneous phase determining section (12R) for successively determining an instantaneous phase $\phi R(f, t)$ of a right electroencephalography signal XR, (ii) a left instantaneous phase determining section (12L) for determining an instantaneous phase $\phi L(f, t)$ of a left electroencephalography signal XL, (iii) a phase synchronization index calculating section (13) for successively calculating a phase synchronization index PSI(f, t), and (iv) a video signal generating section (14) for generating a video signal representing a video image whose content changes in correspondence with the phase synchronization index PSI (f, t).

FIG. 1

## Description

Technical Field

The present invention relates to an electroencephalography (EEG) signal processing device and an EEG signal processing method each for processing EEG signals. The present invention further relates to (i) a program for causing a computer to function as such an EEG signal processing device and (ii) a recording medium on which such a program is stored.

Background Art

Individual parts of the brain show respective activities, which may be regarded as oscillators, and a higher function of the brain may be regarded as a phenomenon of phase synchronization between those oscillators. This can be confirmed by, for example, alternately displaying for a subject as an animation (i) a first image showing a ball at an upper left portion of the screen and another ball at a lower right portion of the screen and (ii) a second image showing a ball at an upper right portion of the screen and another ball at a lower left portion of the screen (see Non Patent Literature 1).

Observing EEGs of a subject viewing those alternating images can actually confirm that the subject sees the two balls as being vertically arranged and horizontally oscillating in phase opposite to each other and that there is phase synchronization between (i) an EEG indicative of activity of the right hemisphere (hereinafter referred to as "right EEG") and (ii) an EEG indicative of activity of the left hemisphere (hereinafter referred to as "left EEG"). This is absolute proof that this phenomenon of phase synchronization directly indicates a higher function of the brain of identifying (i) the ball (recognized by the right hemisphere) at a upper left portion of the screen in the first image with (ii) the ball (recognized by the left hemisphere) at an upper right portion of the screen in the second image.

Observing EEGs of a stroke patient can confirm something similar: Observing EEGs of a stroke patient who suffers a disorder in higher functions of the brain shows that phase synchronization between a right EEG and a left EEG is more difficult to achieve for the stroke patient than for a healthy individual. Further, there has been reported a research result that there is a significant correlation between (i) how easily phase synchronization is achieved between a right EEG and left EEG of a stroke patient immediately after the stroke and (ii) how effective rehabilitation is (Non Patent Literature 2).

Citation List

Non Patent Literature 1

Michael Rose and one other, "Neural Coupling Binds Visual Tokens to Moving Stimuli", The Journal of Neuroscience, November 2, 2005, 25(44), pp. 10101-10104

Non Patent Literature 2

Wenqing Wu and six others, "Impaired neuronal synchrony after focal ischemic stroke in elderly patients", Clinical Neurophysiology, EL SEVIER, published online on June 29, 2010, 122(2011), pp. 21-26

Summary of Invention

Technical Problem

The research result reported in Non Patent Literature 2 suggests that rehabilitation of a stroke patient may produce an increased effect in a case where the stroke patient is placed in a state in which phase synchronization between a right EEG and a left EEG is easy to achieve.

However, no method has been known that allows a stroke patient to train his/her brain to achieve the above state. Further, no method has been known that allows an operation performer to train the brain of a stroke patient for him/her to achieve the above state.

The present invention has been accomplished in view of the above problem. It is an object of the present invention to provide an EEG signal processing device usable in training that facilitates phase synchronization between a right EEG and a left EEG.

The object of the present invention is not limited to increasing the effect of rehabilitation of a stroke patient. Facilitating phase synchronization between a right EEG and a left EEG not only increases the effect of rehabilitation of a stroke patient, but also can contribute to increasing the effect of rehabilitation of a brain disease patient in general. Such facilitation can also contribute to improving higher functions of the brain of a healthy individual.

Solution to Problem

In order to attain the above object, an electroencephalography signal processing device of the present invention includes: an instantaneous phase determining section for successively determining (i) a right instantaneous phase from a right electroencephalography signal indicative of an electroencephalography of a right hemisphere of a subject, the right instantaneous phase being an instantaneous phase of the right electroencephalography signal, and (ii) a left instantaneous phase from a left electroencephalography signal indicative of an electroencephalography of a left hemisphere of the subject, the left instantaneous phase being an instantaneous phase of the left electroencephalography signal; a phase synchronization index calculating section for successively calculating, from the right instantaneous phase and the left instantaneous phase each successively determined by the instantaneous phase determining section, a phase synchronization index indicative of a degree of phase synchronization between the right electroencephalography signal and the left electroencephalography signal; and a signal generating section for generating, on a basis of the phase synchronization index successively calculated by the phase synchronization index calculating section, a signal to be supplied to a stimulus providing device in order to provide the subject with a stimulus that changes in correspondence with the phase synchronization index.

In order to attain the above object, an electroencephalography signal processing method of the present invention includes the steps of: (a) successively determining (i) a right instantaneous phase from a right electroencephalography signal indicative of an electroencephalography of a right hemisphere of a subject, the right instantaneous phase being an instantaneous phase of the right electroencephalography signal, and (ii) a left instantaneous phase from a left electroencephalography signal indicative of an electroencephalography of a left hemisphere of the subject, the left instantaneous phase being an instantaneous phase of the left electroencephalography signal; (b) successively calculating, from the right instantaneous phase and the left instantaneous phase each successively determined in the step (a), a phase synchronization index indicative of a degree of phase synchronization between the right electroencephalography signal and the left electroencephalography signal; and (c) generating, on a basis of the phase synchronization index successively calculated in the step (b), a signal to be supplied to a stimulus providing device in order to provide the subject with a stimulus that changes in correspondence with the phase synchronization index.

Advantageous Effects of Invention

The present invention allows a subject to train his/her brain to facilitate the phase synchronization between the right EEG and the left EEG.

Brief Description of Drawings

Fig. 1 is a block diagram illustrating a configuration of an EEG signal processing device of an embodiment of the present invention.
Fig. 2 is a block diagram illustrating a variation of the EEG signal processing device illustrated in Fig. 1.
Fig. 3 shows diagrams each illustrating a video image displayed by the EEG signal processing device illustrated in Fig. 1.

Fig. 4 is a block diagram illustrating a configuration of a computer that functions as the EEG signal processing device illustrated in Fig. 1.

Description of Embodiments

The description below deals with an electroencephalography (EEG) signal processing device of an embodiment of the present invention with reference to the drawings. The description below uses the expression "substantially equal" about two amounts to mean that those two amounts share the same order of magnitude, in other words, that the larger of the two amounts is greater than the smaller by a factor of less than 10.

[Arrangement of EEG Signal Processing Device]

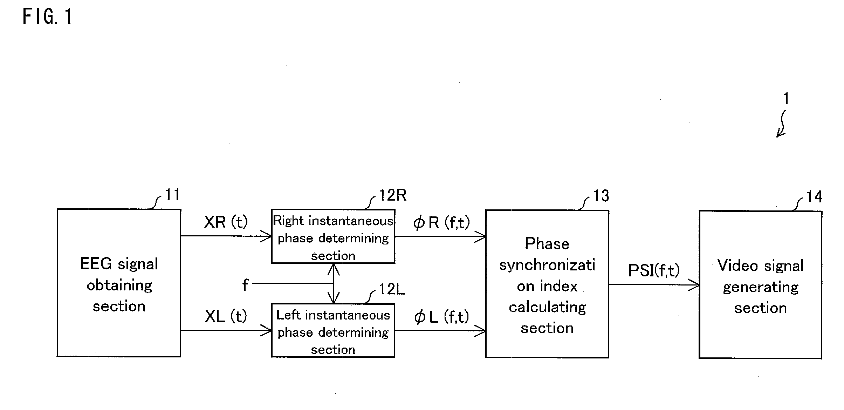

The description below deals with an arrangement of an EEG signal processing device 1 of the present embodiment with reference to Fig. 1.

Fig. 1 is a block diagram illustrating a configuration of the EEG signal processing device 1. The EEG signal processing device 1, as illustrated in Fig. 1, includes an EEG signal obtaining section 11, a right instantaneous phase determining section 12R, a left instantaneous phase determining section 12L, a phase synchronization index calculating section 13, and a video signal generating section 14.

The EEG signal obtaining section 11 obtains, from an external device (for example, an electroencephalograph) connected to the EEG signal processing device 1, (i) an EEG signal XR indicative of an EEG of the right hemisphere of a subject and (ii) an EEG signal XL indicative of an EEG of the left hemisphere of the subject. The EEG signal obtaining section 11 transmits the EEG signal XR and EEG signal XL that it has obtained to the right instantaneous phase determining section 12R and the left instantaneous phase determining section 12L, respectively.

The EEG signal XR is supplied from a source such as an electrode attached to the right hemisphere of the head of the subject. In this case, the EEG signal obtaining section 11 obtains an EEG signal XR indicative of an EEG of the right hemisphere of the subject by sampling, at a predetermined sampling cycle $\Delta t$, respective electric potentials of the electrode which electric potentials are detected by the external device. The sampling cycle $\Delta t$ is, for example, not shorter than 1 millisecond and not longer than 4 milliseconds (in the present embodiment, $\Delta t = 1$ millisecond). The description below uses the expression "XR(t)" to refer to the value of an EEG signal XR sampled at a time point t.

The EEG signal XL is supplied from a source such as an electrode attached to the left hemisphere of the head of the subject. In this case, the EEG signal obtaining section 11 obtains an EEG signal XL indicative of an EEG of the left hemisphere of the subject by sampling, at the above sampling cycle $\Delta t$, respective electric potentials of the electrode which electric potentials are detected by the external device. The description below uses the expression "XL(t)" to refer to the value of an EEG signal XL sampled at a time point t.

The right instantaneous phase determining section 12R determines an instantaneous phase φR(f, t) successively (in real time) from the EEG signal XR. The instantaneous phase φR(f, t) refers to the phase at a time point t of, among component waves included in the EEG signal XR, a component wave XRf having a target frequency f. This target frequency f is specified by an operator, for example. In a case where, for instance, the target frequency f is 10 Hz, the right instantaneous phase determining section 12R determines an instantaneous phase φR(f, t) of an α wave of the right hemisphere of the subject. The right instantaneous phase determining section 12R transmits information on the value of the instantaneous phase φR(f, t) that it has determined to the phase synchronization index calculating section 13 as illustrated in Fig. 1.

The left instantaneous phase determining section 12L determines an instantaneous phase φL(f, t) successively from the EEG signal XR. The instantaneous phase φL(f, t) refers to the phase at a time point t of, among component waves included in the EEG signal XL, a component wave XLf having a target frequency f. The left instantaneous phase determining section 12L transmits information on the value of the instantaneous phase φL(f, t) that it has determined to the phase synchronization index calculating section 13 as illustrated in Fig. 1.

The right instantaneous phase determining section 12R may determine the instantaneous phase φR(f, t) through a publicly known method such as convolution involving a Morlet wavelet. The right instantaneous phase determining section 12R, in a case where it performs convolution involving a Morlet wavelet, determines the instantaneous phase φR(f, t) with reference to respective values XR(t1),..., and XR(tM) of M EEG signals XR sampled at respective sampling timings ti between a time point t1 (= t - Δt × (M-1)/2) and a time point tM (= t + Δt × (M-1)/2) (for the sake of simplicity, M is assumed to be an odd number). The EEG signal processing device 1, for that operation, includes a buffer (not shown) for storing respective values of at least M EEG signals XR. The EEG signal obtaining section 11 supplies EEG signals XR to the right instantaneous phase determining section 12R through that buffer. The description above in this paragraph applies also to how the left instantaneous phase determining section 12L determines the instantaneous phase φL(f, t).

Assuming that the right instantaneous phase determining section 12R determines the instantaneous phase φR(f, t) within a window width T (tM - t1 = Δt × (M-1)) and that the right instantaneous phase determining section 12R requires a calculation time period τ to calculate the instantaneous phase φR(f, t) from XR(t1),..., and XR(tM), at least a time period T/2 + τ is needed between (i) completion of the sampling of an EEG signal XR(t) and (ii) completion of the determination of the instantaneous phase φR(f, t). The window width T is typically not shorter than 3 times and not longer than 8 times a cycle (1/f) corresponding to the target frequency f. Thus, in a case where the target frequency f is, for example, 20 Hz, the window width T is not shorter than 150 milliseconds and not longer than 400 milliseconds.

The present embodiment is arranged such that the right instantaneous phase determining section 12R determines the instantaneous phase φR(f, t) in a minimally required time period T/2 + τ. In other words, the present embodiment is arranged such that between (i) completion of the sampling of the value XR(t) of an EEG signal XR and (ii) completion of the determination of the instantaneous phase φR(f, t), there is a delay δ of a minimally required time period T/2 + τ. The delay δ between (i) completion of the sampling of the value XR(t) of an EEG signal XR and (ii) completion of the determination of the instantaneous phase φR(f, t) simply needs to be substantially equal to the minimally required time period T/2 + τ for sufficiently attaining the object of the present invention. The present specification describes the instantaneous phase φR(f, t) as being determined successively (in real time) to mean that the delay δ is substantially equal to τ + T/2. The description above in this paragraph applies also to how the left instantaneous phase determining section 12L determines the instantaneous phase φL(f, t).

The phase synchronization index calculating section 13 calculates a phase synchronization index PSI(f, t) successively (in real time) from an instantaneous phase φR(f, t) and an instantaneous phase φL(f, t). The phase synchronization index

PSI(f, t) refers to a value that serves as an indicator of the degree of phase synchronization between an EEG signal XL (more precisely, a component wave XLf thereof) and an EEG signal XR (more precisely, a component wave XRf thereof) at a time point t. The phase synchronization index calculating section 13 transmits information on the value of the phase synchronization index PSI(f, t) that it has calculated to the video signal generating section 14 as illustrated in Fig. 1.

The present embodiment is arranged such that the phase synchronization index calculating section 13, to calculate the phase synchronization index PSI(f, t) defined by the equation below, refers to N pairs (for the sake of simplicity, N is assumed to be an odd number) of instantaneous phases {φR(f, t'1), φL(f, t'1)},..., and {φR(f, t'N), φL(f, t'N)} corresponding to respective sampling timings t'i between a time point t'1 (t - Δt × (N - 1)/2) and a time point t'N (= t + Δt × (N - 1)/2). In the equation below, θi(f, t'i) refers to a phase difference defined by θi(f, t'i) = φR(f, t'i) - φL(f, t'i).

[Math. 1]

$$PSI(f,t) = \frac{1}{N} \sum_{i=1}^{N} e^{i\theta(f, t1)}$$

With the phase synchronization index PSI(f, t) defined as above, PSI(f, t) = 1 in a case where the phase of an EEG signal XR perfectly synchronizes with the phase of an EEG signal XL at a time point t, and PSI(f, t) = 0 in a case where the phase of an EEG signal XR does not synchronize at all with the phase of an EEG signal XL at a time point t. The phase synchronization index PSI(f, t) defined as above can thus serve as an appropriate indicator of the degree of phase synchronization between an EEG signal XR and an EEG signal XL.

As described above, the phase synchronization index calculating section 13, to calculate the phase synchronization index PSI(f, t) at a time point t, refers to N pairs of instantaneous phases $\{\phi R(f, t'1), \phi L(f, t'1)\},...,$ and $\{\phi R(f, t'N), \phi L(f, t'N)\}$ at respective sampling timings t'i between a time point t'1 (= t - $\Delta$t $\times$ (N - 1)/2) and a time point t'N (= t + $\Delta$t $\times$ (N - 1)/2). The EEG signal processing device 1, for that operation, includes a buffer (not shown) for storing respective values of at least N instantaneous phases $\phi R(f, t)$. The right instantaneous phase determining section 12R supplies instantaneous phases $\phi L(f, t)$ to the phase synchronization index calculating section 13 through that buffer. The EEG signal processing device 1, for a similar reason, includes a buffer (not shown) for storing respective values of at least N instantaneous phases $\phi L(f, t)$. The left instantaneous phase determining section 12L supplies instantaneous phases $\phi L(f, t)$ to the phase synchronization index calculating section 13 through that buffer.

Assuming that the phase synchronization index calculating section 13 calculates the phase synchronization index PSI(f, t) within a window width T' (t'N - t'1 = $\Delta$t $\times$ (N - 1)) and that the phase synchronization index calculating section 13 requires a time period $\tau$' to calculate the phase synchronization index PSI(f, t) from $\{\phi R(f, t'1), \phi L(f, t'1)\},...,$ and $\{\phi R(f, t'N), \phi L(f, t'N)\}$, at least a time period T'/2 + $\tau$' is needed between (i) completion of the determination of an instantaneous phase $\phi R(f, t)$ and $\phi L(f, t)$ and (ii) completion of the calculation of the phase synchronization index PSI(f, t). The window width T' is typically not shorter than 3 times and not longer than 8 times a cycle (1/f) corresponding to the target frequency f. Thus, in a case where the target frequency f is, for example, 20 Hz, the window width T' is not shorter than 150 milliseconds and not longer than 400 milliseconds.

The present embodiment is arranged such that the phase synchronization index calculating section 13 calculates the phase synchronization index PSI(f, t) in a minimally required time period T/2 + $\tau$. In other words, the present embodiment is arranged such that between (i) completion of the determination of an instantaneous phase $\phi R(f, t)$ and $\phi L(f, t)$ and (ii) completion of the calculation of the phase synchronization index PSI(f, t), there is a delay $\delta$' of a minimally required time period T'/2 + $\tau$'. The delay $\delta$' between (i) completion of the determination of an instantaneous phase $\phi R(f, t)$ and $\phi L(f, t)$ and (ii) completion of the calculation of the phase synchronization index PSI(f, t) simply needs to be substantially equal to the minimally required time period T'/2 + $\tau$' for sufficiently attaining the object of the present invention. The present specification describes the phase synchronization index PSI(f, t) as being calculated successively (in real time) to mean that the delay $\delta$' is substantially equal to the minimally required time period T'/2 + $\tau$'.

The phase synchronization index calculating section 13 may calculate the phase synchronization index PSI(f, t) at $\Delta$t (sampling cycle), at T' (window width), or at T'/2. In a case where the phase synchronization index calculating section 13 calculates the phase synchronization index PSI(f, t) at T' or T'/2, the EEG signal processing device 1 has a reduced calculation load.

The video signal generating section 14 generates, from the phase synchronization index PSI(f, t), a video signal representing a video image whose content changes in correspondence with the phase synchronization index PSI(f, t). The video signal generating section 14 supplies the video signal that it has generated to a display connected to or built in the EEG signal processing device 1. The content of a video image represented by a video signal generated by the video signal generating section 14 will be described later with reference to a different drawing.

The display connected to or built in the EEG signal processing device 1 displays for the subject the video image represented by a video signal generated by the video signal generating section 14, that is, the video image whose content changes in correspondence with the phase synchronization index PSI(f, t). The display, stated differently, functions as a stimulus providing device for providing the subject with a visual stimulus that changes in correspondence with the phase synchronization index PSI(f, t). This forms a real-time feedback loop that allows a subject to receive a visual stimulus as feedback in response to the current state of synchronization between the right hemisphere and left hemisphere of the subject.

The EEG signal processing device 1 is arranged such that between (i) completion of the sampling of the value XR(t) or XL(t) of an EEG signal XR or XL and (ii) completion of the determination of an instantaneous phase $\phi R(f, t)$ or $\phi L(f, t)$, there is a delay $\delta$ of a minimally required time period T/2 + $\tau$. The EEG signal processing device 1 is further arranged such that between (i) completion of the determination of an instantaneous phase $\phi R(f, t)$ and $\phi L(f, t)$ and (ii) completion of the calculation of the phase synchronization index PSI(f, t), there is a delay $\delta$' of a minimally required time period T'/2 + $\tau$'. This indicates that in disregard of a time period required for generation and output of a video signal, there is, between (i) occurrence of a change in the degree of phase synchronization between the EEG signal XR and the EEG signal XL and (ii) reflection of that change in the video image displayed for the subject, a delay (hereinafter referred to as "response time period of the EEG signal processing device 1") of T/2 + $\tau$ + T'/2 + $\tau$' (in a case where the instantaneous phase $\phi R(f, t)$ and the instan-

taneous phase $\phi L(f, t)$ are determined serially, $T/2 + 2 \times \tau + T'/2 + \tau'$). Assuming, for example, that the window widths T and T' are both 400 milliseconds (T = T' = 400 milliseconds), in a case where the calculation time periods $\tau$ and $\tau'$ are sufficiently smaller respectively than the window widths T and T', the response time period of the EEG signal processing device 1 is approximately 400 milliseconds.

In a case where the response time period of the EEG signal processing device 1 is not longer than five seconds, the subject can learn about the degree of phase synchronization between his/her EEGs substantially in real time. This in turn allows the subject to control the degree of phase synchronization between his/her EEGs at will. Receiving feedback in real time means that the response time period of the EEG signal processing device 1 is not longer than five seconds. A shorter response time period of the EEG signal processing device 1 allows feedback to be provided for the subject more immediately, allowing the subject to easily control the degree of phase synchronization between his/her brains at will. The response time period of the EEG signal processing device 1 may be set to, for example, not longer than four seconds, not longer than three seconds, not longer than two seconds, or not longer than one second, among which a shorter time period produces a greater effect.

[Variation]

The EEG signal processing device 1 of Fig. 1 is arranged to determine instantaneous phases $\phi R(f, t)$ and $\phi L(f, t)$ through convolution involving a Morlet wavelet. The present invention is, however, not limited to such an arrangement. The EEG signal processing device 1 may alternatively be arranged to determine instantaneous phases $\phi R(f, t)$ and $\phi L(f, t)$ by, for example, (i) passing EEG signals through a frequency filter and then (ii) performing Hilbert transform on the filtered EEG signals to generate analytic signals.

The EEG signal processing device 1 of Fig. 1 is arranged to calculate a phase synchronization index $PSI(f, t)$ from respective instantaneous phases $\phi R(f, t)$ and $\phi L(f, t)$ of component waves XRf and XLf included respectively in EEG signals XR and XL and each having a particular frequency. The present invention is, however, not limited to such an arrangement. The EEG signal processing device 1 may alternatively be arranged to calculate a phase synchronization index $PSI(t)$ from respective instantaneous phases $\phi R(t)$ and $\phi L(t)$ of EEG signals XR and XL themselves each including all frequency components. Note that the term "instantaneous phase" of an EEG signal as used in the claims of the present application may refer to either (i) an instantaneous phase $\phi R(f, t)$ or $\phi L(f, t)$ or (ii) an instantaneous phase $\phi R(t)$ or $\phi L(t)$, and is not limited to the former.

The EEG signal processing device 1 of Fig. 1 is arranged to generate a video signal to be supplied to a stimulus providing device (for example, a display device) for providing a subject with a visual stimulus that changes in correspondence with the phase synchronization index $PSI(f, t)$. The present invention is, however, not limited to such an arrangement. The EEG signal processing device 1 may alternatively be replaced with any device arranged to generate a signal to be supplied to a stimulus providing device for providing a subject with a stimulus that changes in correspondence with the phase synchronization index $PSI(f, t)$.

The EEG signal processing device 1 may be replaced with, for example, a device for generating an audio signal to be supplied to a stimulus providing device for providing a subject with an auditory stimulus that changes in correspondence with the phase synchronization index $PSI(f, t)$. Examples of such a stimulus providing device for providing a subject with an auditory stimulus include a loudspeaker. The stimulus providing device may provide a subject with an auditory stimulus having (i) a volume that changes in correspondence with the phase synchronization index $PSI(f, t)$, (ii) a musical interval that changes in correspondence with the phase synchronization index $PSI(f, t)$, or (iii) a tone color that changes in correspondence with the phase synchronization index $PSI(f, t)$. In other words, the EEG signal processing device 1 may generate an audio signal having (i) an amplitude that changes in correspondence with the phase synchronization index $PSI(f, t)$, (ii) a cycle that changes in correspondence with the phase synchronization index $PSI(f, t)$, or (iii) a waveform that changes in correspondence with the phase synchronization index $PSI(f, t)$.

The EEG signal processing device 1 may also be replaced with a device for generating a signal to be supplied to a stimulus providing device for providing a subject with a somatic sensation stimulus that changes in correspondence with the phase synchronization index $PSI(f, t)$. Examples of such a stimulus providing device for providing a subject with a somatic sensation stimulus include (i) an electric stimulator for stimulating a somatic sensation of a subject by an electric means and (ii) a mechanical stimulator for stimulating a somatic sensation of a subject by a mechanical means. An example of such a mechanical stimulator is a rehabilitation gear for bending a wrist. The stimulus providing device may provide a subject with a somatic sensation stimulus having (i) a strength that changes in correspondence with the phase synchronization index $PSI(f, t)$, (ii) a cycle that changes in correspondence with the phase synchronization index $PSI(f, t)$, or (iii) a pattern that changes in correspondence with the phase synchronization index $PSI(f, t)$. In other words, the EEG signal processing device 1 may generate a signal having (i) an amplitude that changes in correspondence with the phase synchronization index $PSI(f, t)$, (ii) a cycle that changes in correspondence with the phase synchronization index $PSI(f, t)$, or (iii) a waveform that changes in correspondence with the phase synchronization index $PSI(f, t)$.

The EEG signal processing device 1 may alternatively be replaced with, other than a device for providing a visual stimulus, an auditory stimulus, or a somatic sensation stimulus each corresponding to the phase synchronization index PSI(f, t), a device for providing an olfactory stimulus or a gustatory stimulus each corresponding to the phase synchronization index PSI(f, t). In short, the object of the present invention is attainable with use of any device that allows the subject to learn about the phase synchronization index PSI(f, t) in the form of a sensation.

The EEG signal processing device 1 of Fig. 1 is arranged to calculate a phase synchronization index PSI(f, t) from a pair of EEG signals {XR, XL}. The present invention is, however, not limited to such an arrangement. The EEG signal processing device 1 may alternatively be arranged to calculate a phase synchronization index PSI(f, t) from n EEG signals {X1, X2,..., and Xn} (where n is an even number 2m). The EEG signals X1,..., and Xn indicate respective electric potentials of electrodes attached to respective positions on the head of the subject which positions are different from each other.

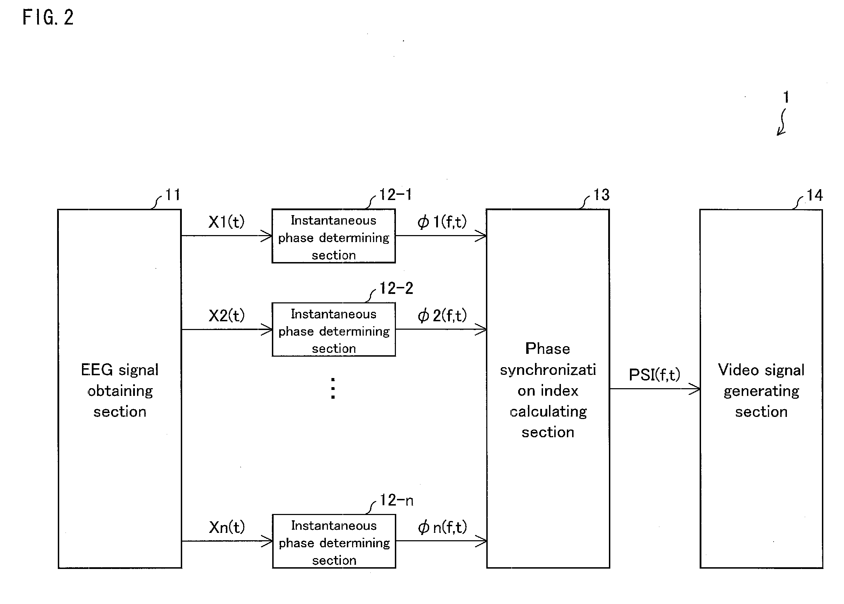

Fig. 2 is a block diagram illustrating a configuration of such an EEG signal processing device 1. The EEG signal processing device 1 of Fig. 2 differs from the EEG signal processing device 1 of Fig. 1 in that it includes n instantaneous phase determining sections 12-1 to 12-n collectively serving functions equivalent to the respective functions of the right instantaneous phase determining section 12R and left instantaneous phase determining section 12L of Fig. 1. The EEG signal processing device 1 of Fig. 2 also differs from the EEG signal processing device 1 of Fig. 1 in that the phase synchronization index calculating section 13 calculates (i) $PSIj(f, t)$ from instantaneous phases $\phi j(f, t)$ and $\phi j+1(f, t)$ for each j (where $1 \leq j \leq m$) and (ii) $\{PSI1(f, t) +...+ PSIm(f, t)\}/m$ (that is, the mean value of $PSIj(f, t)$) as a phase synchronization index PSI(f, t). Except for these differences, the EEG signal processing device 1 of Fig. 2 is arranged identically to the EEG signal processing device 1 of Fig. 1.

The EEG signal processing device 1 of Fig. 2 is arranged to calculate: a phase synchronization index PSI1(f, t) from a pair of an EEG signal X1 and an EEG signal X2; a phase synchronization index PSI2 (f, t) from a pair of an EEG signal X3 and an EEG signal X4;...; and a phase synchronization index PSIm(f, t) from a pair of an EEG signal Xn-1 and an EEG signal Xn. The present invention is, however, not limited to such an arrangement. The EEG signal processing device 1 may be arranged to calculate a phase synchronization index from any pair of EEG signals out of n $\times$ (n - 1)/2 possible pairs, as long as at least one of those pairs is of (i) an EEG signal indicative of the electric potential of an electrode attached to a position on the right hemisphere of the head of the subject and (ii) an EEG signal indicative of the electric potential of an electrode attached to a position on the left hemisphere of the head of the subject.

[Video Image Displayed for Subject]

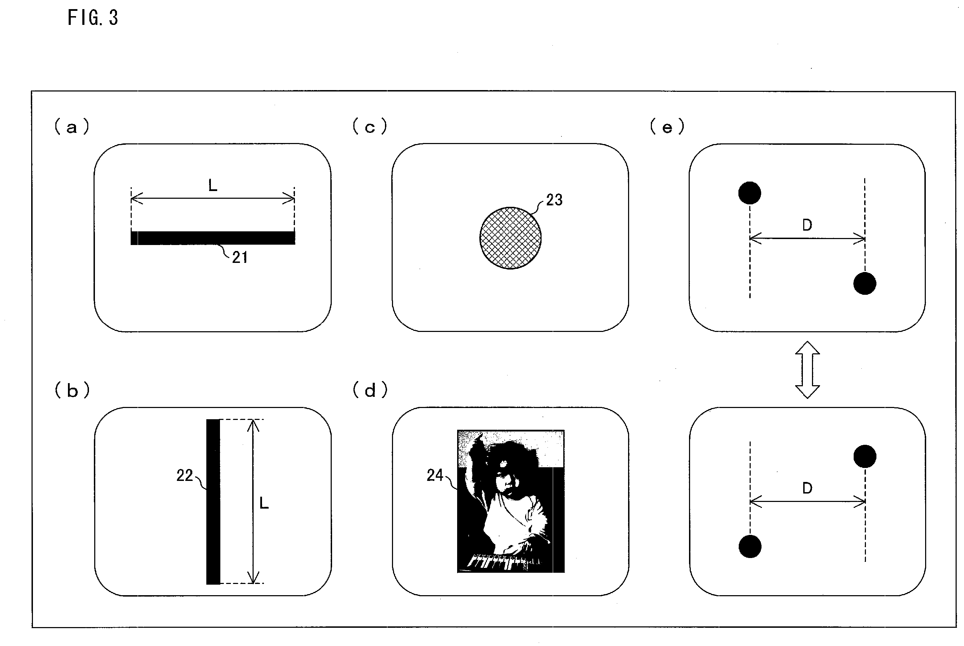

With reference to Fig. 3, the description below deals with example video images displayed by the EEG signal processing device 1 for a subject. (a) through (e) of Fig. 3 are each a diagram illustrating an example video image displayed by the EEG signal processing device 1 for a subject.

The video image illustrated in (a) of Fig. 3 shows, at the center of the screen, a rectangular object 21 having long sides parallel to the vertical axis of the screen. The object 21 in this video image has long sides whose length L changes in correspondence with the phase synchronization PSI(f, t). The length L may change in correspondence with the phase synchronization PSI(f, t) in any manner. In this example, the object 21 has long sides whose length L is positively correlated with the phase synchronization index PSI(f, t). More specifically, the object 21 has long sides whose length L is directly proportional to the phase synchronization index PSI(f, t).

In a case where this video image is displayed for a subject, the subject is tasked with trying to psychokinetically extend the object 21 along its long sides for an increased length L. The subject, in this case, tries to psychokinetically extend the object 21 along its long sides for an increased length L in order to control his/her brain for promotion of synchronization between the right hemisphere and the left hemisphere.

The video image illustrated in (b) of Fig. 3 shows, at the center of the screen, a rectangular object 22 having long sides parallel to the horizontal axis of the screen. The object 22 in this video image has long sides whose length L changes in correspondence with the phase synchronization PSI(f, t). The length L may change in correspondence with the phase synchronization PSI(f, t) in any manner. In this example, the object 22 has long sides whose length L is positively correlated with the phase synchronization index PSI(f, t). More specifically, the object 22 has long sides whose length L is directly proportional to the phase synchronization index PSI(f, t).

In a case where this video image is displayed for a subject, the subject is tasked with trying to psychokinetically extend the object 22 along its long sides for an increased length L. The subject, in this case, tries to psychokinetically extend the object 22 along its long sides for an increased length L in order to control his/her brain for promotion of synchronization between the right hemisphere and the left hemisphere.

The video image illustrated in (c) of Fig. 3 shows, at the center of the screen, a colored, disk-shaped object 23 having a variable color temperature. The object 23 in this video image has a color temperature that changes in correspondence with the phase synchronization PSI(f, t). The color temperature may change in correspondence with the phase synchronization PSI(f, t) in any manner. In this example, the object 23 has a color temperature that is negatively correlated with the phase synchronization index PSI(f, t). More specifically, the object 23 has a color temperature that is inversely proportional to the phase synchronization index PSI(f, t).

In a case where this video image is displayed for a subject, the subject is tasked with trying to psychokinetically warm the color (decreasing the temperature) of the object 23. The subject, in this case, tries to psychokinetically warm the color of the object 23 in order to control his/her brain for promotion of synchronization between the right hemisphere and the left hemisphere.

The video image illustrated in (d) of Fig. 3 shows, at the center of the screen, a gray-scale photograph 24 whose number of gray levels is variable ((d) of Fig. 3 shows, as an example, a gray-scale photograph having two gray levels, that is, a monochrome photograph). The gray-scale photograph 24 in this video image has gray levels whose number changes in correspondence with the phase synchronization PSI(f, t) within the range of 2 to 256. The number of gray levels may change in correspondence with the phase synchronization PSI(f, t) in any manner. In this example, the photograph 24 has gray levels whose number is positively correlated with the phase synchronization index PSI(f, t). More specifically, the photograph 24 has gray levels whose number is directly proportional to the phase synchronization index PSI(f, t).

In a case where this video image is displayed for a subject, the subject is tasked with, for example, identifying the object. The subject, in this case, tries to psychokinetically increase the number of gray levels for the photograph 24 and identify the object in order to control his/her brain for promotion of synchronization between the right hemisphere and the left hemisphere.

The video image illustrated in (e) of Fig. 3 is an animation that alternately displays (i) an image in the upper half of (e) of Fig. 3 which image shows two balls, one at an upper left portion and the other at a lower right portion, and (ii) an image in the lower half of (e) of Fig. 3 which image shows two balls, one at an upper right portion and the other at a lower left portion. The two balls in this animation are apart from each other along the horizontal axis of the screen by a distance d that changes in correspondence with the phase synchronization index PSI(f, t). The distance d may change in correspondence with the phase synchronization index PSI(f, t) in any manner. In this example, the two balls are apart from each other by a distance d that is negatively correlated with the phase synchronization index PSI(f, t).

This animation may be recognized in one of the two manners: (i) two horizontally arranged balls vertically oscillate in phase opposite to each other, or (ii) two vertically arranged balls horizontally oscillate in phase opposite to each other. In a case where a subject recognizes the animation in the latter manner, the respective phases of the right hemisphere and left hemisphere of the subject likely synchronize with each other. Further, in a case where the respective phases of the right hemisphere and left hemisphere of a subject synchronize with each other, the subject likely recognizes the animation in the latter manner. This is because if a subject recognizes the animation in the latter manner, it directly means that the subject allows his/her right hemisphere and left hemisphere to cooperate with each other to identify (i) a ball at an upper left portion of the image illustrated in the upper half of (e) of Fig. 3 with (ii) a ball at an upper right portion of the image illustrated in the lower half of (e) of Fig. 3.

In a case where this animation is displayed for a subject, the subject receives a question such as "Do you see the two balls as being vertically arranged and horizontally oscillating in phase opposite to each other?". The subject tries to recognize the animation in that manner in order to control his/her brain for promotion of synchronization between the right hemisphere and the left hemisphere.

[Example Configuration of EEG Signal Processing Device]

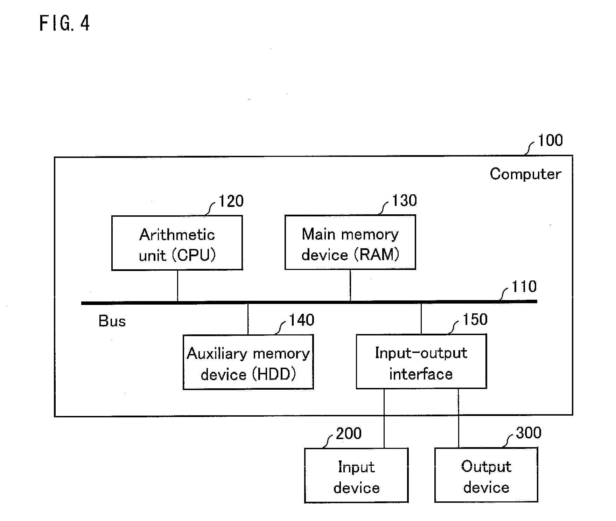

The EEG signal processing device 1 may be in the form of, for example, a computer (electronic calculator). Fig. 4 is a block diagram illustrating an example configuration of a computer 100 usable as the EEG signal processing device 1.

The computer 100, as illustrated in Fig. 4, includes an arithmetic unit 120, a main memory device 130, an auxiliary memory device 140, and an input-output interface 150, which are all connected to each other via a bus 110. Example devices usable as the arithmetic unit 120 include a central processing unit (CPU). Example devices usable as the main memory device 130 include a semiconductor random access memory (RAM). Example devices usable as the auxiliary memory device 140 include a hard disk drive.

The input-output interface 150 is, as illustrated in Fig. 4, connected to an input device 200 and an output device 300. Examples of the input device 200 connected to the input-output interface 150 include (i) an electroencephalograph for detecting EEG signals XR and XL and (ii) a keyboard for specifying a target frequency f. Examples of the output device 300 connected to the input-output interface 150 include a display for displaying a video image whose content changes in correspondence with the phase synchronization index PSI(f, t).

The auxiliary memory device 140 stores various programs for causing the computer 100 to operate as the EEG signal processing device 1. Specifically, the auxiliary memory device 140 stores an EEG signal obtaining program, a right instantaneous phase determining program, a left instantaneous phase determining program, a phase synchronization index calculating program, and a video signal generating program. These programs may each be a module included in a numerical calculation library such as MATLAB (registered trademark).

The arithmetic unit 120 loads the above programs, stored in the auxiliary memory device 140, into the main memory device 130 and executes the instructions written in the programs loaded in the main memory device 130. The arithmetic unit 120 thus causes the computer 100 to function as the EEG signal obtaining section 11, the right instantaneous phase determining section 12R, the left instantaneous phase determining section 12L, the phase synchronization index calculating section 13, and the video signal generating section 14. The main memory device 130 functions also as (i) a buffer for storing the value of an EEG signal XR and as (ii) a buffer for storing instantaneous phases $\phi R(f, t)$ and $\phi L(f, t)$.

The present embodiment described herein is arranged to cause the computer 100 to function as the EEG signal processing device 1 with use of the programs stored on an internal recording medium (auxiliary memory device 140). The present invention is, however, not limited to such an arrangement. The present embodiment may be arranged to cause the computer 100 to function as the EEG signal processing device 1 with use of programs stored on an external recording medium. Examples of such an external recording medium include a computer-readable non-transitory tangible medium such as a tape, a disk, a card, a semiconductor memory, and a programmable logic circuit.

The present embodiment may alternatively be arranged such that the computer 100 is capable of connecting to a communication network and receives the above programs over the communication network. The communication network may be any communication network that allows transmission of programs. The present invention may further be in the form of a data signal embedded in a carrier wave and embodied by electronic transmission of the programs.

[Applications of EEG Signal Processing Device]

The EEG signal processing device 1 is, as an example, used for rehabilitation of a patient with a brain disease such as stroke.

A stroke patient tends to have a phase synchronization index PSI lower than that of a healthy individual. This is believed to reflect unsmooth cooperation between the right hemisphere and left hemisphere of a stroke patient. A stroke patient tends to have a decreased phase synchronization index PSI regardless of the site damaged by the stroke. It is known, for instance, that a stroke patient has a decreased phase synchronization index PSI even if the damaged site is part of the right hemisphere.

Recent research has revealed about rehabilitation of stroke patients that the phase synchronization index PSI measured before the start of rehabilitation is correlated with the effect of the rehabilitation. Specifically, the research has revealed that rehabilitation tends to be more effective for a stroke patient having a high phase synchronization index PSI than for a stroke patient having a low phase synchronization index PSI.

This tendency suggests that rehabilitation of a stroke patient may produce an increased effect in a case where the rehabilitation incorporates training for increasing the phase synchronization index PSI. The EEG signal processing device 1 may thus be used as a training device for such training. It is needless to say that a similar effect is expected to be produced even in a case where the EEG signal processing device 1 is used for rehabilitation of a patient with a brain disease other than stroke.

[Recap]

As described above, in order to attain the above object, an electroencephalography signal processing device of the present embodiment includes: an instantaneous phase determining section for successively determining (i) a right instantaneous phase from a right electroencephalography signal indicative of an electroencephalography of a right hemisphere of a subject, the right instantaneous phase being an instantaneous phase of the right electroencephalography signal, and (ii) a left instantaneous phase from a left electroencephalography signal indicative of an electroencephalography of a left hemisphere of the subject, the left instantaneous phase being an instantaneous phase of the left electroencephalography signal; a phase synchronization index calculating section for successively calculating, from the right instantaneous phase and the left instantaneous phase each successively determined by the instantaneous phase determining section, a phase synchronization index indicative of a degree of phase synchronization between the right electroencephalography signal and the left electroencephalography signal; and a signal generating section for generating, on a basis of the phase synchronization index successively calculated by the phase synchronization index calculating section, a signal to be supplied to a stimulus providing device in order to provide the subject with a stimulus that changes in correspondence with the phase synchronization index.

The above arrangement allows the subject to receive, in real time, feedback of the degree of the phase synchronization between the right EEG and left EEG of the subject. The above arrangement thus allows the subject to control his/her brain at will in order to increase the degree of the phase synchronization between the right EEG and the left EEG. The subject can repeat such control to train his/her brain in order to facilitate the phase synchronization between the right EEG and the left EEG.

The electroencephalography signal processing device of the present embodiment may preferably be arranged such that in a case where the degree of the phase synchronization between the right electroencephalography signal and the left electroencephalography signal has changed, the stimulus provided by the stimulus providing device for the subject reflects the change in the degree not longer than five seconds after the change in the degree.

The above arrangement allows the subject to receive the feedback more immediately, and can thus increase the effect of the training.

The electroencephalography signal processing device of the present embodiment may preferably be arranged such that between (i) completion of sampling of respective values of the right electroencephalography signal and the left electroencephalography signal each for a time point t and (ii) completion of the determination of the right instantaneous phase and the left instantaneous phase each for the time point t, there is a delay substantially equal to $T/2 + \tau$ or $T/2 + 2 \times \tau$, where T represents a window width for use by the instantaneous phase determining section to determine the right instantaneous phase and the left instantaneous phase, and $\tau$ represents a calculation time period required by the instantaneous phase determining section to calculate either of the right instantaneous phase and the left instantaneous phase.

The above arrangement can shorten the response time between (i) occurrence of a change in the degree of the phase synchronization between the right EEG and the left EEG and (ii) reflection of that change in information presented to the subject. In other words, the above arrangement allows the subject to receive the feedback more immediately, and can thus increase the effect of the training.

The electroencephalography signal processing device of the present embodiment may preferably be arranged such that between (i) completion of the determination of the right instantaneous phase and the left instantaneous phase each for a time point t and (ii) completion of the calculation of the phase synchronization index for the time point t, there is a delay substantially equal to $T'/2 + \tau'$, where T' represents a window width for use by the phase synchronization index calculating section to calculate the phase synchronization index, and $\tau'$ represents a calculation time period required by the phase synchronization index calculating section to calculate the phase synchronization index.

The above arrangement can shorten the response time between (i) occurrence of a change in the degree of the phase synchronization between the right EEG and the left EEG and (ii) reflection of that change in information presented to the subject. In other words, the above arrangement allows the subject to receive the feedback more immediately, and can thus increase the effect of the training.

The electroencephalography signal processing device of the present embodiment may preferably be arranged such that the stimulus providing device is a display device; and the signal generating section generates a video signal to be supplied to the display device in order to provide the subject with a visual stimulus that changes in correspondence with the phase synchronization index.

The above arrangement allows the subject to receive a visual stimulus (video image) as feedback of the degree of the phase synchronization between the right EEG and left EEG of the subject.

The electroencephalography signal processing device of the present embodiment may preferably be arranged such that the stimulus providing device is a loudspeaker; and the signal generating section generates an audio signal to be supplied to the loudspeaker in order to provide the subject with an auditory stimulus that changes in correspondence with the phase synchronization index.

The above arrangement allows the subject to receive an auditory stimulus (sound) as feedback of the degree of the phase synchronization between the right EEG and left EEG of the subject.

The electroencephalography signal processing device of the present embodiment may preferably be arranged such that the stimulus providing device is an electric stimulator or mechanical stimulator; and the signal generating section generates a signal to be supplied to the electric stimulator or mechanical stimulator in order to provide the subject with a somatic sensation stimulus that changes in correspondence with the phase synchronization index.

The above arrangement allows the subject to receive a somatic sensation stimulus as feedback of the degree of the phase synchronization between the right EEG and left EEG of the subject.

The electroencephalography signal processing device of the present embodiment may be arranged such that the electroencephalography signal processing device functions as a training device for use by a brain disease patient to train a brain of the brain disease patient in order to facilitate phase synchronization between a right electroencephalography and a left electroencephalography.

Using the electroencephalography signal processing device of the present embodiment as the above training device can increase the effect of rehabilitation of the brain disease patient.

As described above, in order to attain the above object, an electroencephalography signal processing method of the present embodiment includes the steps of: (a) successively determining (i) a right instantaneous phase from a right electroencephalography signal indicative of an electroencephalography of a right hemisphere of a subject, the right instantaneous phase being an instantaneous phase of the right electroencephalography signal, and (ii) a left instantaneous phase from a left electroencephalography signal indicative of an electroencephalography of a left hemisphere of the subject, the left instantaneous phase being an instantaneous phase of the left electroencephalography signal; (b) successively calculating, from the right instantaneous phase and the left instantaneous phase each successively determined in the step (a), a phase synchronization index indicative of a degree of phase synchronization between the right electroencephalography signal and the left electroencephalography signal; and (c) generating, on a basis of the phase synchronization index successively calculated in the step (b), a signal to be supplied to a stimulus providing device in order to provide the subject with a stimulus that changes in correspondence with the phase synchronization index.

The above arrangement allows the subject to receive, in real time, feedback of the degree of the phase synchronization between the right EEG and left EEG of the subject. The above arrangement thus allows the subject to control his/her brain at will in order to increase the degree of the phase synchronization between the right EEG and the left EEG. The subject can repeat such control to train his/her brain in order to facilitate the phase synchronization between the right EEG and the left EEG.

The electroencephalography signal processing device of the present embodiment may be in the form of a computer. In that case, the present invention covers in its scope (i) a program for causing a computer to operate as each of the sections of the electroencephalography signal processing device in order to cause the computer to function as the electroencephalography signal processing device and (ii) a computer-readable recording medium on which that program is stored.

[Supplemental Notes]

The present invention is not limited to the description of the embodiments above, but may be altered in various ways by a skilled person within the scope of the claims. Any embodiment based on a proper combination of technical means disclosed in different embodiments is also encompassed in the technical scope of the present invention. Further, combining technical means disclosed in different embodiments can provide a new technical feature.

Industrial Applicability

The EEG signal processing device of the present invention is suitably usable as, for example, a training device for increasing the effect of rehabilitation of a brain disease patient. The EEG signal processing device is further usable generally for applications to improve a higher function of the brain. The EEG signal processing device is also usable as, for example, a game device.

Reference Signs List

1 EEG signal processing device
11 EEG signal obtaining section
12R right instantaneous phase determining section (instantaneous phase determining section)
12L left instantaneous phase determining section (instantaneous phase determining section)
13 phase synchronization index calculating section
14 video signal generating section
100 computer
140 auxiliary memory device (recording medium)
XR EEG signal (right EEG signal)
XL EEG signal (left EEG signal)

$\phi R(f, t)$ instantaneous phase (right instantaneous phase)
$\phi L(f, t)$ instantaneous phase (left instantaneous phase)
PSI(f, t) phase synchronization index

**Claims**

1. An electroencephalography signal processing device, comprising:

an instantaneous phase determining section for successively determining (i) a right instantaneous phase from a right electroencephalography signal indicative of an electroencephalography of a right hemisphere of a subject, the right instantaneous phase being an instantaneous phase of the right electroencephalography signal, and (ii) a left instantaneous phase from a left electroencephalography signal indicative of an electroencephalography of a left hemisphere of the subject, the left instantaneous phase being an instantaneous phase of the left electroencephalography signal;
a phase synchronization index calculating section for successively calculating, from the right instantaneous phase and the left instantaneous phase each successively determined by the instantaneous phase determining section, a phase synchronization index indicative of a degree of phase synchronization between the right electroencephalography signal and the left electroencephalography signal; and
a signal generating section for generating, on a basis of the phase synchronization index successively calculated by the phase synchronization index calculating section, a signal to be supplied to a stimulus providing device in order to provide the subject with a stimulus that changes in correspondence with the phase synchronization index.

2. The electroencephalography signal processing device according to claim 1,
wherein:
in a case where the degree of the phase synchronization between the right electroencephalography signal and the left electroencephalography signal has changed, the stimulus provided by the stimulus providing device for the subject reflects the change in the degree not longer than five seconds after the change in the degree.

3. The electroencephalography signal processing device according to claim 1 or 2,
wherein
between (i) completion of sampling of respective values of the right electroencephalography signal and the left electroencephalography signal each for a time point t and (ii) completion of the determination of the right instantaneous phase and the left instantaneous phase each for the time point t, there is a delay substantially equal to $T/2 + \tau$ or $T/2 + 2 \times \tau$, where T represents a window width for use by the instantaneous phase determining section to determine the right instantaneous phase and the left instantaneous phase, and $\tau$ represents a calculation time period required by the instantaneous phase determining section to calculate either of the right instantaneous phase and the left instantaneous phase.

4. The electroencephalography signal processing device according to claim 1 or 2,
   wherein
   between (i) completion of the determination of the right instantaneous phase and the left instantaneous phase each for a time point t and (ii) completion of the calculation of the phase synchronization index for the time point t, there is a delay substantially equal to $T'/2 + \tau'$, where T' represents a window width for use by the phase synchronization index calculating section to calculate the phase synchronization index, and $\tau'$ represents a calculation time period required by the phase synchronization index calculating section to calculate the phase synchronization index.

5. The electroencephalography signal processing device according to any one of claims 1 to 4,
   wherein:

   the stimulus providing device is a display device; and
   the signal generating section generates a video signal to be supplied to the display device in order to provide the subject with a visual stimulus that changes in correspondence with the phase synchronization index.

6. The electroencephalography signal processing device according to any one of claims 1 to 4,
   wherein:
   the stimulus providing device is a loudspeaker; and
   the signal generating section generates an audio signal to be supplied to the loudspeaker in order to provide the subject with an auditory stimulus that changes in correspondence with the phase synchronization index.

7. The electroencephalography signal processing device according to any one of claims 1 to 4,
   wherein:

   the stimulus providing device is an electric stimulator or mechanical stimulator; and

the signal generating section generates a signal to be supplied to the electric stimulator or mechanical stimulator in order to provide the subject with a somatic sensation stimulus that changes in correspondence with the phase synchronization index.

8. The electroencephalography signal processing device according to any one of claims 1 to 7,
   wherein
   the electroencephalography signal processing device functions as a training device for use by a brain disease patient to train a brain of the brain disease patient in order to facilitate phase synchronization between a right electroencephalography and a left electroencephalography.

9. An electroencephalography signal processing method, comprising the steps of:

   (a) successively determining (i) a right instantaneous phase from a right electroencephalography signal indicative of an electroencephalography of a right hemisphere of a subject, the right instantaneous phase being an instantaneous phase of the right electroencephalography signal, and (ii) a left instantaneous phase from a left electroencephalography signal indicative of an electroencephalography of a left hemisphere of the subject, the left instantaneous phase being an instantaneous phase of the left electroencephalography signal;
   (b) successively calculating, from the right instantaneous phase and the left instantaneous phase each successively determined in the step (a), a phase synchronization index indicative of a degree of phase synchronization between the right electroencephalography signal and the left electroencephalography signal; and
   (c) generating, on a basis of the phase synchronization index successively calculated in the step (b), a signal to be supplied to a stimulus providing device in order to provide the subject with a stimulus that changes in correspondence with the phase synchronization index.

10. A program for causing a computer to function as an electroencephalography signal processing device according to any one of claims 1 to 8, the program causing the computer to function as each of the sections.

11. A computer-readable recording medium on which a program according to claim 10 is stored.

FIG. 1

FIG. 2

FIG. 3

(a)

(b)

(c)

(d)

(e)

EP 2 962 635 A1

FIG. 4

**INTERNATIONAL SEARCH REPORT**

| International application No. |
| --- |
| PCT/JP2014/054559 |

**A. CLASSIFICATION OF SUBJECT MATTER**
*A61B5/0484*(2006.01)i

According to International Patent Classification (IPC) or to both national classification and IPC

**B. FIELDS SEARCHED**

Minimum documentation searched (classification system followed by classification symbols)
A61B5/0484, A61M21/02, A61B10/00

Documentation searched other than minimum documentation to the extent that such documents are included in the fields searched
Jitsuyo Shinan Koho       1922-1996   Jitsuyo Shinan Toroku Koho   1996-2014
Kokai Jitsuyo Shinan Koho  1971-2014   Toroku Jitsuyo Shinan Koho   1994-2014

Electronic data base consulted during the international search (name of data base and, where practicable, search terms used)
JSTPlus(JDreamIII), JMEDPlus(JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 63-97148 A (Matsushita Electric Works, Ltd.), 27 April 1988 (27.04.1988), entire text; all drawings (Family: none) | 1-8,10-11 |
| Y | WO 2011/155465 A1 (Saburo UEMORI), 15 December 2011 (15.12.2011), paragraphs [0048], [0049] (Family: none) | 1-8,10-11 |
| A | JP 2006-110234 A (Riken, Japan), 27 April 2006 (27.04.2006), entire text; all drawings (Family: none) | 1-8,10-11 |

☒ Further documents are listed in the continuation of Box C.     ☐ See patent family annex.

| | |
| --- | --- |
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search <br> 23 April, 2014 (23.04.14) | Date of mailing of the international search report <br> 13 May, 2014 (13.05.14) |
| --- | --- |
| Name and mailing address of the ISA/ <br> Japanese Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2014/054559 |

C (Continuation).  DOCUMENTS CONSIDERED TO BE RELEVANT

| Category* | Citation of document, with indication, where appropriate, of the relevant passages | Relevant to claim No. |
| --- | --- | --- |
| A | JP 2008-529743 A  (Aspect Medical Systems, Inc.),<br>07 August 2008 (07.08.2008),<br>entire text; all drawings<br>& US 2005/0216071 A1    & US 2005/0043774 A1<br>& EP 1850742 A1          & WO 2006/089181 A1 | 1-8,10-11 |
| A | JP 2012-170815 A  (Kazumasa SHIGA),<br>10 September 2012 (10.09.2012),<br>entire text; all drawings<br>(Family: none) | 1-8,10-11 |

Form PCT/ISA/210 (continuation of second sheet) (July 2009)

<table>
<tr><td colspan="2">**INTERNATIONAL SEARCH REPORT**</td><td>International application No.<br>PCT/JP2014/054559</td></tr>
</table>

| **Box No. II** | **Observations where certain claims were found unsearchable (Continuation of item 2 of first sheet)** |
|---|---|

This international search report has not been established in respect of certain claims under Article 17(2)(a) for the following reasons:

1. ☒ Claims Nos.: 9
   because they relate to subject matter not required to be searched by this Authority, namely:
   Claim 9 pertains to a method for treatment of the human body or animal body by therapy and thus relates to a subject matter on which this International Searching Authority is not required to carry out an international search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| **Box No. III** | **Observations where unity of invention is lacking (Continuation of item 3 of first sheet)** |
|---|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MICHAEL ROSE.** Neural Coupling Binds Visual Tokens to Moving Stimuli. *The Journal of Neuroscience,* 02 November 2005, vol. 25 (44), 10101-10104

- Impaired neuronal synchrony after focalischemic stroke in elderly patients. **WENQING WU.** Clinical Neurophysiology. EL SEVIER, 29 June 2010, vol. 122, 21-26